# EUROPEAN PATENT APPLICATION

(11) **EP 2 095 782 A1**
(43) Date of publication of application: **02.09.2009**
(21) Application number: 08012418.3
(22) Date of filing: 09.07.2008
(51) Int. Cl.: A61B 17/70

(54) **Buffer type vertebral pedicle screw**

(30) Priority: 27.02.2008 KR 20080017872
(71) Applicant: Choi, Gil-Woon, Yonsu-gu Incheon 406-120 (KR)
(72) Inventor: Choi, Gil-Woon, Yonsu-gu Incheon 406-120 (KR)
(74) Representative: Müller-Boré & Partner Patentanwälte

(57) **Abstract**

A vertebral pedicle screw includes a screw body driven into a vertebral pedicle, a housing assembled to an upperend of the screw body and having grooves for seating a rod and a threaded portion for locking a fastening bolt, the fastening bolt pressing the rod positioned thereunder as it is threaded downward, and a fastening ring inserted into the housing. The vertebral pedicle screw further includes an insert placed on an outer surface of a screw head of the screw body in the housing, installed to be partially come into contact with the fastening ring on an upper portion thereof, and accommodating the screw head of the screw body on a curved inner surface thereof such that the insert and the screw head are brought into sliding contact with each other, whereby an entire rod-side assembly can be slidingly moved in all directions with respect to the screw body.

## Description

### [Technical Field]

The present invention relates to a vertebral pedicle screw used when conducting spine surgery in the field of orthopedics, and more particularly, to a buffer type vertebral pedicle screw which can absorb a shock using a relative sliding characteristic between a screw body driven into a vertebral pedicle and a rod, thereby relieving the pain of a patient and ensuring the stabilization of abody part receiving a surgical operation.

### [Background Art]

In general, a patient having the damaged spine cannot lead an active life. Even though the patient can be active to some extent due to the lesser degree of the damage to the spine, as the damaged portion of the spine is pressed by or brought into contact with an adjacent portion, a pain is caused to the patient, and a recovery is likely to be retarded.

Therefore, for the patient having the damaged spine, a surgical operationis conducted to support the adjacent portion of the spine using artificial prosthetics so that the damaged portion of the spine is not pressed by the adjacent portion.

In this case, the artificial prosthetics for supporting the spine is composed of a plurality of vertebral pedicle screws and a rod. The vertebral pedicle screws are driven into vertebral pedicles over and under a damaged vertebral pedicle and serve as fasteners, and the rod is securely held by the plurality of vertebral pedicle screws and serves as a support.

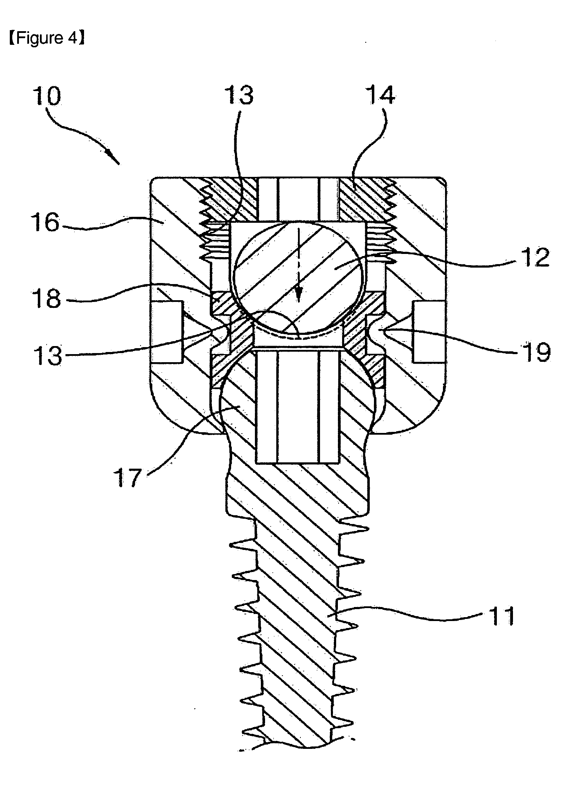

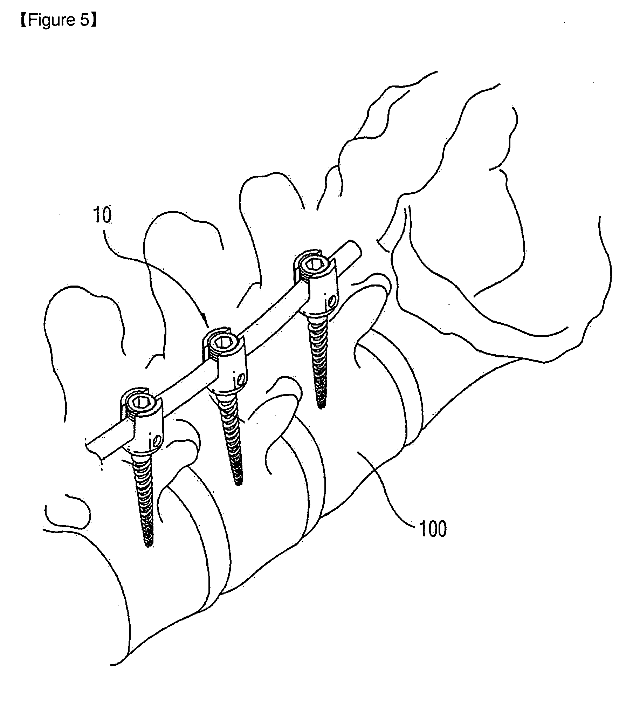

FIG. 4 is a sectional view illustrating the assembled state of a conventional vertebral pedicle screw, and FIG. 5 is a perspective view illustrating the use of the conventional vertebral pedicle screw.

A vertebral pedicle screw 10 has a screw body 11 which is driven into a vertebral pedicle 100, a housing 16 which is assembled to the upper portion of the screw body 11 and has U-shaped grooves 13 for placing a rod 12 therein and a threaded portion 15 to be locked with a fastening bolt 14, the fastening bolt 14 being locked to the threaded portion 15 of the housing 16 to press the rod 12 positioned thereunder as it is threaded downwards, and a fastening ring 18 which is disposed in the housing 16 to press a screw head 17 of the screw body 11.

The unexplained reference numeral 19 designates punched portions for fixing the position of the fastening ring 18.

A procedure for connecting vertebral pedicles 100 using vertebral pedicle screws 10 and the rod 12 will be described below.

First, screw bodies 11 of the vertebral pedicle screws 10 are vertically driven into the respective vertebral pedicles 100 which are to be connected with one another. Then, fastening rings 18 are respectively inserted into housings 16 of the vertebral pedicle screws 10, and the rod 12 is placed in the U-shaped grooves 13 of the housings 16.

In this state, by locking respective fastening bolts 14 into threaded portions 15 of the housings 16, as the lower surfaces of the fastening bolts 14 press the rod 12, the rod 12 which connects the vertebral pedicles 100 with one another is securely held.

In succession, by punching the housings 16 on both sides of the respective fastening rings 18 which press the screw heads 17 on the lower ends thereof and support the rod 12 on the upper ends thereof and thereby forming punched portions 19, the surgical operation for the spine using the vertebral pedicle screws 10 and the rod 12 is completed.

However, in the conventional vertebral pedicle screw 10, since the screw body 11 and the fastening ring 18 are coupled with each other such that they are secured tight to each other and are not moved relative to each other, that is, such that the fastening ring 18 firmly grasps the screw head 17 of the screw body 11, a problem is caused in that the body part receiving the surgical operation can be unstabilized even by slight motion of a patient.

For example, as the shock or vibration transferred to the screw body 11 through the rod 12 due to the motion of the patient is applied as it is, to the entire body part receiving the surgical operation, a pain is caused in the body part receiving the surgical operation, which acts as a factor impeding the stabilization of the body part receiving the surgical operation. As a result, inconvenience is rendered to the patient due to the pain, and the recovery of the patient is likely to be retarded.

### [Disclosure]

### [Technical Problem]

Accordingly, the present invention has been made in an effort to solve the problems occurring in the related art, and an object of the present invention is to provide a vertebral pedicle screw, which can realize a novel configuration of a vertebral pedicle screw by allowing a screw body driven into and fastened to a vertebral pedicle and a rod-side assembly to be slidingly moved relative to each other in all directions within a predetermined angle, so that, when a shock is applied, the shock can be absorbed through the relative sliding movement between the screw body and the rod-side assembly, thereby relieving the pain of a patient and ensuring the stabilization of the body part receiving a surgical operation, and the entire surgical operation including a rod connection task can be easily carried out due to the slidable structure of the rod-side assembly, thereby enabling spine surgery to be effectively conducted in terms of time and precision.

### [Technical Solution]

In order to achieve the above object, according to the present invention, there is provided a vertebral pedicle screw including a screw body driven into a vertebral pedicle, a housing assembled to an upper end of the screw body and having U-shaped grooves for seating a rod and a threaded portion for locking a fastening bolt, the fastening bolt locked to the threaded portion of the housing to press the rod positioned thereunder as it is threaded downward, and a fastening ring inserted into the housing, the vertebral pedicle screw comprising an insert placed on an outer surface of a screw head of the screw body in the housing, installed to be partially come into contact with the fastening ring on an upper portion thereof, and accommodating the screw head of the screw body on a curved inner surface thereof such that the insert and the screw head are brought into sliding contact with each other, whereby an entire rod-side assembly can be slidingly moved in all directions with respect to the screw body driven into the vertebral pedicle.

### [Advantageous Effects]

Thanks to the above features, the vertebral pedicle screw according to the present invention provides advantages in that, since a slidable structure between a screw body and a rod-side assembly is adopted, when a shock is applied, the entire rod-side assembly can slidingly move in all directions within a predetermined angle and can thereby absorb or reduce the shock so that the pain of a patient can be relieved to the maximum degree, and it is possible to prevent a shock or vibration from being applied to the vertebral pedicle into which the screw body is driven so that the body part receiving a surgical operation can be kept stabilized.

Also, when conducting a surgical operation for the spine, because the rod-side assembly can slidingly move to some extent depending upon the status of the operation, the entire surgical operation including a rod connection task can be easily carried out so that the surgical operation for the spine can be effectively conducted in terms of time and precision.

As a result, the overall efficiency associated with the surgical operation for the spine can be increased, whereby it is possible to be of help to the operation and the medical treatment of a patient.

### [Description of Drawings]

The above objects, and other features and advantages of the present invention will become more apparent after a reading of the following detailed description taken in conjunction with the drawings, in which:
FIG. 1 is a perspective view illustrating the exploded state of a buffer type vertebral pedicle screw in accordance with an embodiment of the present invention
FIG. 2 is a sectional view illustrating the assembled state of the buffer type vertebral pedicle screw in accordance with the embodiment of the present invention;
FIG. 3 is a sectional view illustrating the sliding characteristic of the buffer type vertebral pediclescrew in accordance with the embodiment of the present invention
FIG. 4 is a sectional view illustrating the assembled state of a conventional vertebral pedicle screw; and
FIG. 5 is a perspective view illustrating the use of the conventional vertebral pedicle screw.

### [Best Mode]

Reference will now be made in greater detail to a preferred embodiment of the invention, an example of which is illustrated in the accompanying drawings. Wherever possible, the same reference numerals will be used throughout the drawings and the description to refer to the same or like parts.

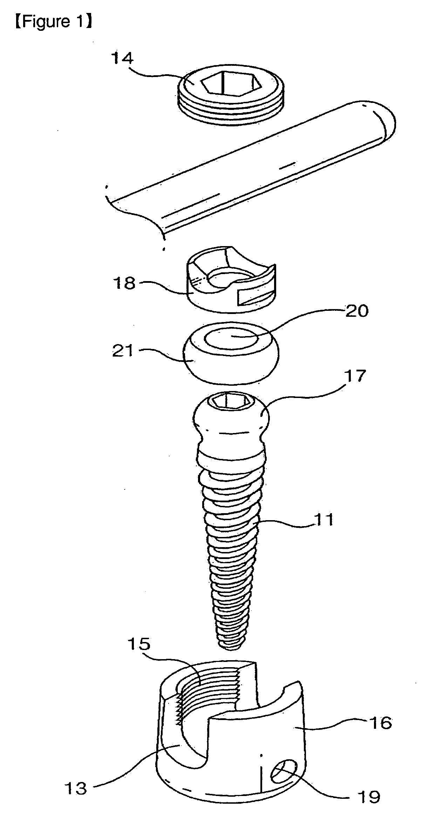

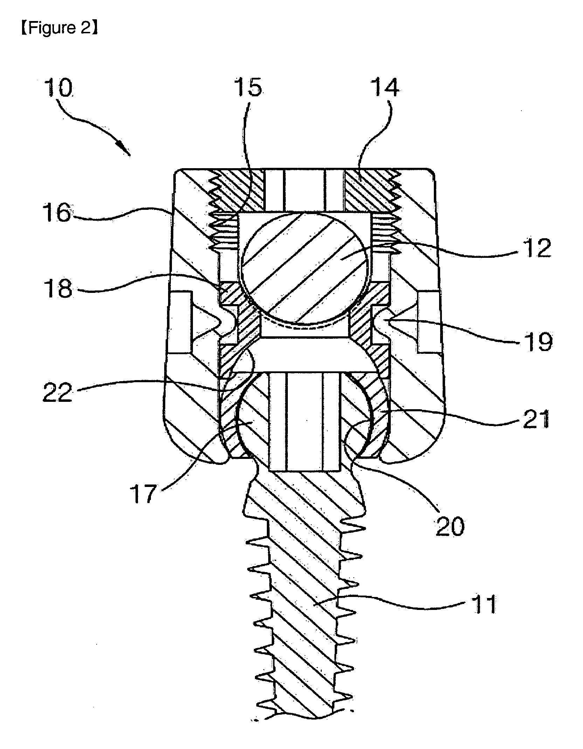

FIG. 1 is a perspective view illustrating the exploded state of a buffer type vertebral pedicle screw in accordance with an embodiment of the present invention, and FIG. 2 is a sectional view illustrating the assembled state of the buffer type vertebral pedicle screw in accordance with the embodiment of the present invention.

Referring to FIGs. 1 and 2, a vertebral pedicle screw 10 in accordance with an embodiment of the present invention is made of titanium(Ti) and serves as a medical subsidiary for securely holding a rod connecting vertebral pedicles with one another.

The vertebral pedicle screw 10 includes a screw body 11 which is driven into each of vertebral pedicles 100 positioned upwardly and downwardly adjacent to the damaged portion of the spine.

The screw body 11 is directly locked to the vertebral pedicle 100 by a threaded portion thereof, and is assembled to a housing 16 via a spherical screw head 17 thereof.

The housing 16 functions to grasp a rod 12. The housing 16 has grooves downwardly extendingfrom the upper end thereof, that is, U-shaped grooves 13, such that the rod 12 can be placed and seated in the U-shaped grooves 13.

A threaded portion 15 is formed on the inner wall surface of the housing 16, and a fastening bolt 14 is locked to the threaded portion 15 of the housing 16. As the fastening bolt 14 is threaded downward, it can press and fasten the rod 12 positioned thereunder.

The vertebral pedicle screw 10 according to the present invention further includes the fastening bolt 14 which is locked to the threaded portion 15 of the housing 16 to press the rod 12, and a fastening ring 18 which is disposed in the housing 16 to stably support the rod 12 placed in the grooves 13 of the housing 16 on the upper end thereof and to come into contact with an insert 21, to be described later, on the lower end thereof.

The fastening ring 18 can be fixedly held at the inserted position by punched portions 19 of the housing 16 which are formed by punching the sidewall of the housing 16 to protrude inward. By forming the punched portions 19 in this way, it is possible to prevent the fastened state of the rod 12 from being released due to the fluctuation of the fastening ring 18.

In particular, the present invention provides a structure for allowing the screw body 11 driven into the vertebral pedicle 100 and a rod-side assembly to be slidingly moved relative to each other.

The rod-side assembly, that is, the entire housing 16 including the fastening bolt 14, the rod 12, the fastening ring 18 and the insert 21 can be slidingly moved with respect to the screw body 11 in all directions within a predetermined angle.

To this end, the insert 21 is provided to surround the circumferential surface of the screw head 17 of the screw body 11. The insert 21 has the sectional shape of a ring. The insert 21 has the configuration of a truncated hollow sphere which has a curved convex outer surface and a curved concave inner surface 20.

The insert 21 is inserted into the housing 16 and is placed at the lower end of the housing 16. The insert 21 is installed such that it surrounds the screw head 17 of the screw body 11 at the lower portion thereof and comes into contact with the lower surface of the fastening ring 18 at the upper portion thereof.

At this time, as the fastening bolt 14 is threaded, the insert 21 can be fixedly held not to be moved, by the rod 12 and the fastening ring 18 which are sequentially pressed by the fastening bolt 14.

The fastening ring 18 has a lower surface which is composed of a curved surface 22, i.e., having the same curvature as the curved outer surface of the insert 21. Since the outer surface of the upper portion of the insert 21 can be partially brought into contact with the curved surface 22 of the fastening ring 18, the insert 21 is fixedly held against movement with respect to the fastening ring 18.

Specifically, the insert 21 has a structure in which the sphericalscrew head 17 of the screw body 11 is accommodated on the curved inner surface 20 of the insert 21 such that the spherical screw head 17 and the insert 21 can be slidingly moved with respect to each other.

According to this, the screw head 17 and the insert 21 can slide with respect to each other in all directions within a predetermined angle. As a result, through the sliding contact between the screw head 17 and the insert 21, an entire rod-side assembly can be moved on the screw body 11 serving as a fixed element.

Here, it is preferred that the angle, within which the rod-side assembly can be moved in all directions, be set to about 10∼15°when measured from the axis of the screw body 11.

A procedure for installing vertebral pedicle screws 10 and thereby fastening the rod 12 when conducting a surgical operation for the spine using the rod 12 and the vertebral pedicle screws 10 according to the present invention, configured as described above, will be described below.

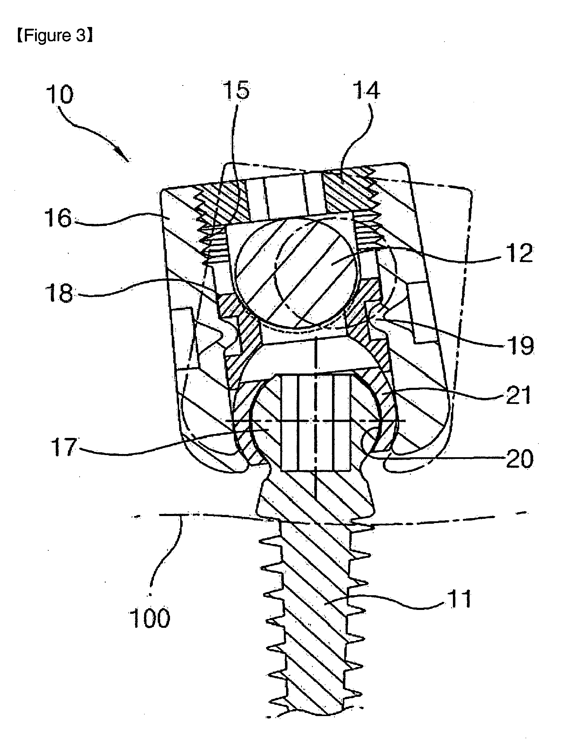

Referring to FIG. 3, first, each screw body 11 having the insert 21 on the screw head 17 thereof is threadedly driven into each of the vertebral pedicles 100 to be connected with one another. Then, after inserting the fastening ring 18 into the housing 16 which is coupled to the screw head 17 of the screw body 11, the rod 12 is fitted into the housing 16.

At this time, the rod 12 is seated in the U-shaped grooves 13 which are defined on both sides of the housing 16.

In this state, the fastening bolt 14 is locked to the threaded portion 15 of the housing 16.

The threading of the fastening nut 14 can be conducted by inserting a tool into the groove defined on the upper end of the fastening bolt 14 and then rotating the tool.

When the fastening bolt 14 is locked in this way, since the rod 12, which is seated on the fastening ring 18 positioned thereunder, is pressed downward by the fastening bolt 14, sufficient and precise pressing force (locking force) can be applied to the rod 12 and the rod 12 can be held in the fastened state. The curved surface 22 of the fastening ring 18, which is positioned under and pressed by the rod 12, is brought into close contact with the outer surface of the upper portion of the insert 21 and fixedly holds the insert 21.

At this time, because a predetermined interval is defined between the screw head 17 of the screw body 11 and the fastening ring 18, the possibility of them to come into contact with each other is eliminated.

In succession, by forming the punched portions 19 by punching the sidewall of the housing 16 on both sides of the fastening ring 18, the fastening ring 18 is secured, by which the installation of the vertebral pedicle screw 10 and the rod 12 is completed.

Accordingly, in the state in which the vertebral pedicle screw is installed, the entire rod-side assemblycan be slidingly moved to some extent within a predetermined angle with respect to the screw body which is driven into the vertebral pedicle, due to the slidingcontact between the screw head and the insert, etc., and as a result, even when a shock is applied to the rod due to the motion of a patient, the shock can be absorbed as the rod-side assembly is slidingly moved, whereby it is possible to minimize the shock transferred to the vertebral pedicle through the screw body.

In the conventional art, due to the fact that the screw body is integrally coupled to the rod-side assembly, the shock is directly transferred from the rod-side assembly through the screw bodyto the vertebral pedicle. In the present invention, unlike the conventional art, since the shock can be absorbed through the sliding movement of the rod-side assembly, the pain of the patient can be relieved to the maximum. Also, due to the absorption of the shock, it is possible to prevent the body part receiving the surgical operation, in particular, where the screw body is driveninto the vertebral pedicle, from being distorted, and thereby, the stabilization of the body part receiving the surgical operation can be ensured.

Also, when placing the rod into the housing after driving the screw body into the vertebral pedicle, since the entire housing including the rod can be adjusted in the angle thereof, the rod placing task can be easily and precisely carried out in a short period, whereby the surgical operation for the spine can be more efficiently conducted.

Although a preferred embodiment of the present invention has been described for illustrative purposes, those skilled in the art will appreciate that various modifications, additions and substitutions are possible, without departing from the scope and spirit of the invention as disclosed in the accompanying claims.

## Claims

1. A vertebral pedicle screw including a screw body driven into a vertebral pedicle, a housing assembled to an upper end of the screw body and having U-shaped grooves for seating a rod and a threaded portion for locking a fastening bolt, the fastening bolt locked to the threaded portion of the housing to press the rod positioned thereunder as it is threaded downward, and a fastening ring inserted into the housing, the vertebral pedicle screw comprising:
an insert placed on an outer surface of a screw head of the screw body in the housing, installed to be partially come into contact with the fastening ring on an upper portion thereof, and accommodating the screw head of the screw body on a curved inner surface thereof such that the insert and the screw head are brought into sliding contact with each other, whereby an entire rod-side assembly can be slidingly moved in all directions with respect to the screw body driven into the vertebral pedicle.

2. The buffer type vertebral pedicle screw according to claim 1, wherein the fastening ring has a curved surface for partially accommodating the upper portion of the insert.

3. The buffer type vertebral pedicle screw according to claim 1 or 2, wherein a movable range of the rod-side assembly is set to 10∼15°in all directions when measured from an axis of the screw body.
